Europäisches Patentamt

European Patent Office

Office européen des brevets

(11) Publication number: **0 167 735**
**A1**

# EUROPEAN PATENT APPLICATION

(21) Application number: **85105147.4**

(22) Date of filing: **26.04.85**

(51) Int. Cl.⁴: **A 61 N 1/05**

(30) Priority: **24.05.84 US 614105**

(43) Date of publication of application:
**15.01.86 Bulletin 86/3**

(84) Designated Contracting States:
**DE FR GB IT NL**

(71) Applicant: **Cordis Corporation**
**10555 West Flagler Street**
**Miami Florida 33102(US)**

(72) Inventor: **Tarjan, Peter P.**
**6951 S.W. 134 Street**
**Miami Florida 33156(US)**

(74) Representative: **KUHNEN & WACKER**
**Patentanwaltsbüro**
**Schneggstrasse 3-5 Postfach 1729**
**D-8050 Freising(DE)**

(54) Atrioventricular pacing/sensing lead.

(57) A distal lead having a distal end, comprising a tubing of flexible elastomeric insulating material having a distal end and a tip electrode (34) and at least one ring electrode (36) spaced rearward of the tip electrode. An elongate rod (38) made of a titanium nickel alloy having heat-triggered mechanical memory properties is initially formed into a shape approaching that of a bell-shaped distribution curve, is annealed in that form and subsequently reformed to a straight shape which the rod has when it is mounted within the distal end portion of the lead for facilitating insertion of the lead distal end portion through an atrium (16) into a ventricle (18). After insertion of the distal end portion of the lead into a ventricle, the lead in the area of the ring electrode is heated to cause the elongate rod to return to its original bell-shaped distribution curve shape to force the ring electrode against the atrial wall for sensing while the tip electrode engages the apex of the ventricle for pacing.

EP 0 167 735 A1

./...

FIG. 3

Cordis Corporation                    56 CO03 14 3/bu

# ATRIOVENTRICULAR PACING/SENSING LEAD

## Field of the Invention

The present invention relates to an atrioventricular pacing/sensing lead which has a generally straight rod of material in the distal end portion thereof that has a mechanical memory such that the rod can be heated to cause the distal end portion to assume a predetermined shape within an atrium and ventricle of a heart to position a tip electrode at the apex of a ventricle for pacing and a ring electrode against the atrial septum for sensing. The rod has an initially straight configuration to facilitate insertion of the distal end portion of the lead through the atrium into the heart.

## Description of the Prior Art

Heretofore it has been proposed in the Berkovits U.S. Patent No. 3,729,008 to provide an electrode for atrial pacing with a curved end for atrial wall attachment which is formed of a resilient catheter body made from a high medical grade durameter rubber that has sufficient flexibility so that it can be bent for insertion through a blood vessel which, upon entering the atrium, will spring back to a J-shaped position for positioning the electrodes in the distal end portion of the lead in the atrium. With this lead, a torque or bending force is exerted by the catheter against

0167735

the wall of blood vessel as it is inserted through the blood vessel into the atrium of the heart.

Also, it has been proposed in the Wilson U.S. Patent No. 3,890,977 to provide a kinetic memory electrode catheter or cannula. The Wilson patent discloses the use of a titanium nickel alloy or titanium-nickel-cobalt alloy that has a mechanical memory which is triggered by heat. In several embodiments, the catheter or cannula is provided with a rod made of the titanium nickel alloy and which, when heated such as by the temperature of the body after the catheter has been inserted through a blood vessel into the heart, will return to a pre-set curved or semi-circular shape where electrodes on the catheter will be positioned at desired locations for atrioventricular pacing.

As will be described in greater detail hereinafter, the atrioventricular pacing/sensing lead of the present invention differs from previously proposed pacing leads and particularly the pacing leads or catheters disclosed in the Wilson U.S. Patent No. 3,890,977 by providing an atrioventricular pacing/sensing lead having an elongate bar or rod of a titanium nickel alloy which, in the cooled state, has a linear or straight configuration for facilitating insertion of the distal end portion of the lead into the atrium and ventricle of the heart and which, when heated, will assume a bell-shaped distribution curve shape whereby a tip electrode of the lead will engage the bottom wall of the ventricle such as for pacing and a ring-shaped electrode spaced rearwardly of the tip electrode is forced to engage the atrial wall of the heart such as for sensing. In this way, sensing and pacing can be achieved with one lead instead of two leads.

## SUMMARY OF THE INVENTION

According to the present invention there is provided an atrioventricular lead having a distal end portion and comprising a tubing of flexible elastomeric insulating material having a distal end, a tip electrode mounted at the distal end of said tubing, a first insulated coiled conductor received within said tubing and having a distal end extending to and being electrically connected to said tip electrode, at least one ring electrode around said tubing spaced rearwardly of said tip electrode, at least one second insulated coiled conductor received within said tubing, surrounding said first insulated coiled conductor and having a distal end which terminates adjacent to, and which is connected to, said ring electrode, and an alongate rod situated within said distal end protion of said lead between said tubing and said second coiled conductor, said rod having a middle portion situated in the area of said ring electrode, a proximal end portion and a distal end portion, said distal end portion of said rod extending toward but not to the location of said tip electrode, said elongate rod being made of a titanium nickel alloy which has a mechanical memory that is triggered by heat, being initially formed with an original shape approaching that of a bell-shaped distribution curve and being annealed in that form and then subsequently reformed to a straight shape which said rod has when it is mounted within said distal end protion of said lead for facilitating insertion of said lead distal end portion into an atrium and a ventricle of a heart whereby after insertion of said tip electrode of said lead into the ventricle said rod in the area of said ring electrode can be heated to cause said elongat rod to return to its original bell-shaped distribution curve shape to force said at least one ring electrode against the atrial wall, such as for sensing, while said tip electrode engages the bottom wall, e.g., apex, of the ventricle, such as for pacing.

### BRIEF DESCRIPTION OF THE DRAWINGS

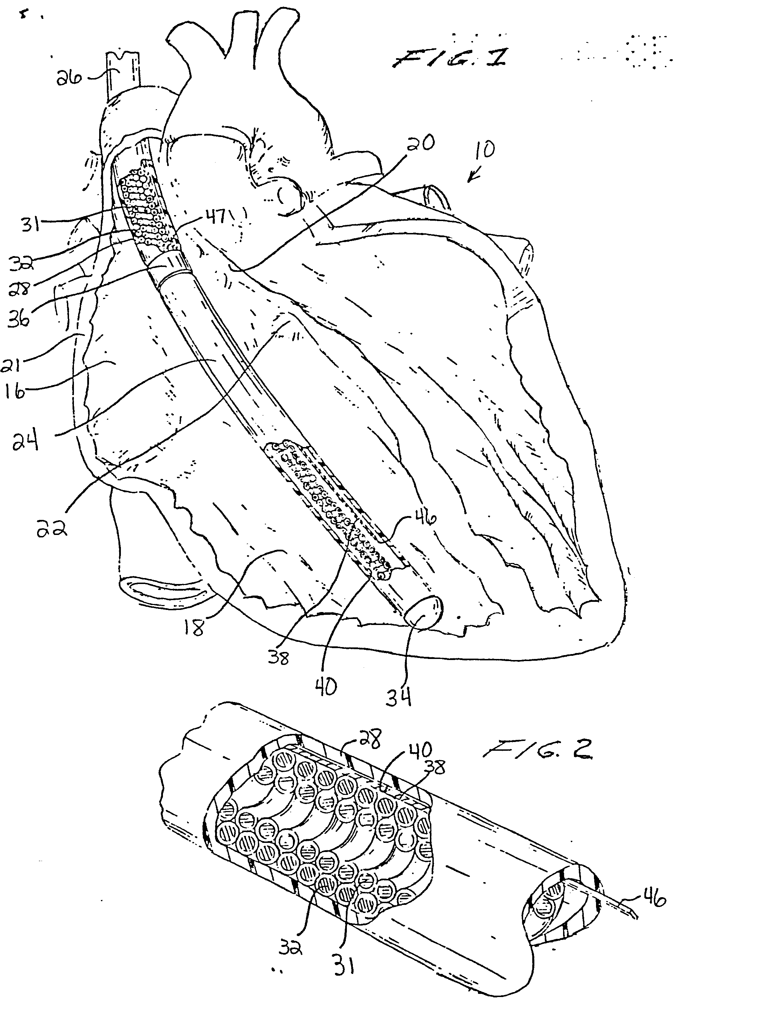

Fig. 1 is an enlarged view of a heart with portion broken away to show the atrium and ventricles of the heart and shows a distal end portion of an atrioventricular pacing lead constructed according to the teachings of the present invention received within the right ventricle and with portions broken away to show interior parts of the lead.

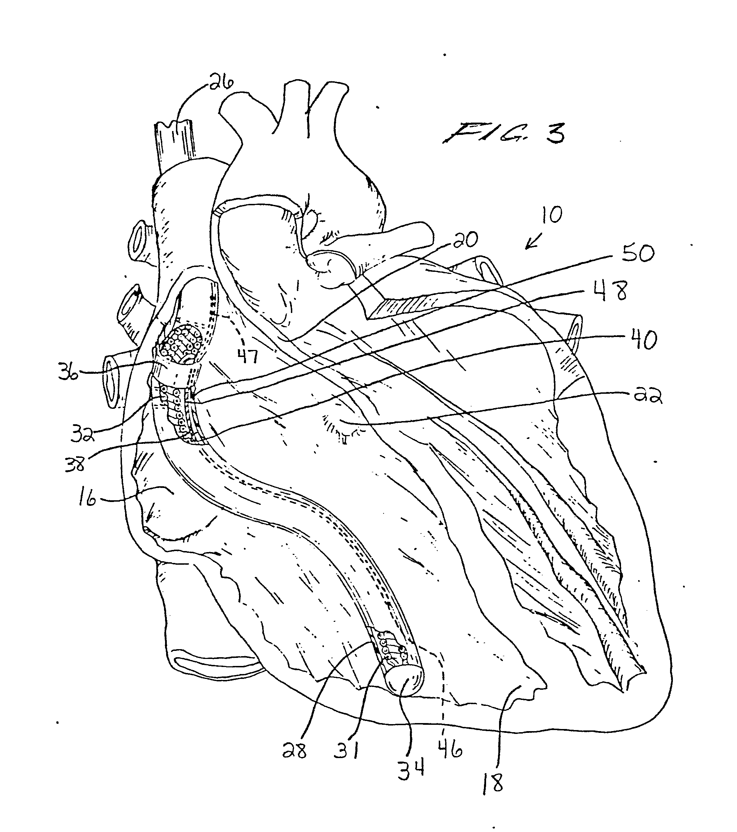

Fig. 2 is a fragmentary perspective view of a portion of the lead shown in Fig. 1.

Fig. 3 is an enlarged perspective view of the heart shown in Fig. 1 after the distal end portion of the lead has been heated to cause an orienting member within the lead to assume a desired shape to place a tip electrode at the bottom of the right ventricle such as for pacing and a ring electrode located rearwardly of the tip electrode adjacent the atrial wall of the heart, such as for sensing.

### DESCRIPTION OF THE PREFERRED EMBODIMENT

Referring now to Fig. 1 there is illustrated therein an enlarged perspective view of a human heart 10 with portions cut away to show the right atrium 16, the right ventricle 18, the atrial septum 20, the atrial wall 21 and the atrioventricular node 22. A distal end portion 24 of a pacing lead 26 of the present invention is received through the right atrium

5

0167735

16 into the right ventricle 18.

The lead 26 includes a sheath, tubing or catheter 28 made of a flexible insulating material such as silicone rubber or polyurethane and first and second insulated coiled conductors 31 and 32 within the lead 26. As shown, the first insulated coiled conductor 31 is received within the second coiled conductor 32 and extends to a tip electrode 34 at the end of the distal end portion 24 of the lead 26. Although hidden from view, it is to be understood that this first insulated coiled conductor 31 is electrically connected to the tip electrode 34.

Then, spaced rearwardly of the tip electrode 34 is a ring electrode 36 which extends around the tubing 28. An end, hidden from view, of the second insulated coiled conductor 32 is connected in a suitable, known manner to the ring electrode 36.

In accordance with the teaching of the present invention, an elongate orienting rod or bar 38 is positioned within the distal end portion 24 of the lead 26 and preferably between the outer or second insulated coiled conductor 32 and inner surface 40 of the sheath or tubing 28. This elongate rod 38 is made of a titanium nickel alloy commonly referred to as Nitinol (Nickel Titanium Naval Ordinance Lab). This alloy can also include traces of cobalt.

Such an alloy has a mechanical memory that is triggered by heat. The alloy is originally formed with restraint into a bell shaped distribution curve shape and heat annealed in that form. Then the rod 38 is deformed at a temperature below its transitional temperature depending upon the relative composition of materials of the alloy into a shape, and in this case a straight shape, facilitating ease of insertion of lead 26 having the rod 38 therein into the body, in this case, the distal end portion 24 of the lead 26, into which the rod 38 is inserted.

Then, after the distal end portion 24 of the lead 26 is inserted through the atrium 16 into the ventricle 18, such as the right ventricle 18 shown in Fig. 1, the lead 25 in the area of the ring electrode 36 is heated to heat the rod 38 to cause the rod 38 to return to the bell-shaped distribution curve shape. The heating can be achieved with microvave radiation. Alternatively, the lead 26 can be heated with a laser by inserting an optic fiber into the lead 26 and then injecting several watts of laser light energy into the lumen of the lead 26. Also the transitional temperature can be at body temperature and the lead 26 can be cooled and then inserted into the heart, after which it slowly heats up and the rod 38 returns to its original shape.

As best shown in Fig. 3, the original shape of the rod 38 is the shape of a typical bell-shaped distribution curve where the ends 46 and 47 of the rod 38 are in line and then the rod 38 curves in the manner shown in Fig. 3 to form a bell-shaped middle portion 48.

Also it will be apparent that the distal end portion 24 of the lead 26 is positioned with the rod 38 on a side 50 of the lead facing the free atrial wall so that when the rod 38 is heated, it causes the distal end portion to take the bell-shaped distribution curve shape 48 shown in Fig. 3 with ring electrode 36 forced against the free atrial wall 21 in

7    0167735

the mid-atrial segment and with the tip electrode 34 engaging the apex of the right ventricle 18.

Typically the ring electrode 36 is used for sensing and the tip electrode 34 is used for pacing. Also, to ensure adequate sensing, several small width ring electrodes can be provided in addition to or in place of ring electrode 36 such as indicated in phantom by reference numerals 61, 62 and 63.

Again the composition of the Nitinol can be such that the transitional temperature is at about the temperature of the human body, e.g., 98-101°F. One preferred composition of the Nitinol is approximately 50 % nickel and approximately 50 % titianium.

From the foregoing description it will be apparent that the atrioventricular pacing/sensing lead 26 of the present invention has a number of advantages, some of which have been described above and others of which are inherent in the invention. In particular, the lead 26 is generally straight so that it can be easily inserted into the atrium 16 and ventricle 18 of a heart 10 after which the rod 38 can be heated above its transitional temperature to cause the rod 38 to resume or return to its original bell-shaped distributuion curve shape 48 such that the tip electrode 34 is urged against the apex of the ventricle for pacing and at the same time ring electrode 36 spaced rearwardly of the tip electrode 34 is forced against the atrial wall 21 in the midatrial segment so that the lead 26 can be utilized for atrial and ventircular sensing and pacing.

Also, it will be appreciated from the foregoing description that modifications can be made to the atrioventricular pacing/sensing lead 26 of the present invention whithout departing from the teachings of the invention. Accordingly the scope of the invention is only to be limited as necessitated by the accompanying claims.

CLAIMS

I Claim:

1. An atrioventricular lead having a distal end portion and comprising a tubing of flexible elastomeric insulating material having a distal end, a tip electrode mounted at the distal end of said tubing, a first insulated coiled conductor received within said tubing and having a distal end extending to and being electrically connected to said tip electrode, at least one ring electrode around said tubing spaced rearwardly of said tip electrode, at least one second insulated coiled conductor received within said tubing, surrounding said first insulated coiled conductor and having a distal end which terminates adjacent to, and which is connected to, said ring electrode and an elongate rod situated within said distal end portion of said lead between said tubing and said second coiled conductor, said rod having a middle portion situated in the area of said ring electrode, a proximal end portion and a distal end portion, said distal end portion of said rod extending toward but not to the location of said tip electrode, said elongate rod being made of a titanium nickel alloy which has a mechanical memory that is triggered by heat, being initially formed with an original shape approaching that of a bell-shaped distribution curve and being annealed in that form and then subsequently reformed to a straight shape which said rod has when it is mounted within said distal end portion of said lead for facilitating insertion of said lead distal end portion into an atrium and a ventricle of a heart whereby after insertion of said tip electrode of said lead into the ventricle, said rod in the area of said ring electrode can be heated to cause said elongate rod to return to its original bell-shaped distribution curve shape to force said at least one ring electrode against the atrial wall such as for sensing while said tip electrode engages the bottom wall, e.g., apex, of the ventricle such as for pacing.

2. The atrioventricular lead of claim 1 including two or more ring electrodes adjacent to but spaced from one another and two or more second conductors, one each for each ring electrode thereby to be able to force several ring electrodes against the atrial wall to enhance good sensing with at least one of said ring electrodes.

3. The atrioventricular lead of claim 2 wherein said ring electrodes have a relatively small width whereby several sensing electrodes can be located close to each other for sensing in a desired atrial wall area.

4. The atrioventricular lead of claim 1 wherein said at least one ring electrode is located on said lead in a position where it will engage the mid-atrial segment.

5. The atrioventricular lead of claim 1 wherein said rod is made of an alloy comprising approximately 50 % titianium and approximately 50 % nickel.

6. The atrioventricular lead of claim 1 wherein said rod is made of a nickel titanium alloy having a transition (memory triggering) temperature between 98 and 101°F.

*FIG. 1*

26

20

10

31

47

32

28

36

21

16

24

22

18

38

40

34

46

*FIG. 2*

28

40

38

32

31

46

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 154 247 (MEDTRONIC INC.) * Column 5, line 61 - column 6, line 32; column 7, lines 26-45; figures 4,5 * | 1 | A 61 N 1/05 |
| A | | 4 | |
| Y,D | US-A-3 890 977 (WILSON) * Column 1, line 43 - column 2, line 24; column 3, line 10 - column 4, line 28 * | 1 | |
| A | | 5,6 | |
| A | WO-A-8 002 801 (REENSTIERNA) * Page 2, lines 7-12; page 3, lines 29-33; page 4, line 17 - page 5, line 2 * | 1-4 | TECHNICAL FIELDS SEARCHED (Int. Cl.4) |
| A | EP-A-0 009 732 (PRECIMED) * Page 3, line 25 - page 4, line 3; page 4, lines 20-35 * | 1 | A 61 N |

The present search report has been drawn up for all claims

| Place of search | Date of completion of the search | Examiner |
|---|---|---|
| THE HAGUE | 22-08-1985 | SIMON J.J.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82